(19) Europäisches Patentamt
European Patent Office
Office européen des brevets

(11) **EP 3 109 782 A1**

(12) **EUROPEAN PATENT APPLICATION**

(43) Date of publication:
28.12.2016 Bulletin 2016/52

(51) Int Cl.:
*G06F 19/00* $^{(2011.01)}$    *G06Q 30/00* $^{(2012.01)}$

(21) Application number: 16170786.4

(22) Date of filing: 23.05.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 08.06.2015 GB 201509916

(71) Applicant: FUJITSU LIMITED
211-8588 Kanagawa (JP)

(72) Inventors:
• HU, Bo
  Winchester, Hampshire SO23 7DT (GB)
• NASEER BUTT, Aisha
  Hayes, Middlesex UB4 8LB (GB)

(74) Representative: Wilding, Frances Ward
Haseltine Lake LLP
Lincoln House, 5th Floor
300 High Holborn
London WC1V 7JH (GB)

(54) **A RISK DETERMINING APPARATUS AND RISK DETERMINING METHOD**

(57) A risk determining apparatus configured to use data to determine a risk of an individual, suffering a mental disorder, taking self-detrimental action, the apparatus comprising: a data input/output module configured to receive data, relating to a focal individual, from a plurality of data sources; a network analysis module configured to determine a social risk component based on the received data; a text analysis module configured to determine an information risk component based on the received data; and a risk aggregator configured to aggregate the social risk component and the information risk component to determine a numeric risk value.

Figure 2

**Description**

The present invention relates to an apparatus and method for assessing the risk of actions being taken by an individual (to the detriment of the individual) suffering from a mental disorder (which is a broad term taken to imply impairment in any of judgment, stability, reliability, or general social or vocational capabilities). The invention may be used alone or in conjunction with a clinician/clinical methods to determine and quantify the likelihood of such individual carrying out such detrimental action, for example, self-harm, based on data relating to the individual.

The invention is in the field of mental health and has applications throughout the world of behavioural analysis.

Background of the Invention

Mental disorders and an individual's susceptibility to them can be influenced by a number of factors including external influences. External influences can be categorised into two categories: societal and environmental (in terms of information exposure), and the risks posed to an individual's health condition can be evaluated through these influences. Examples of these influences include information exposure through social media or other open platforms. However, such social influences need to be quantified.

To consider a specific example, self-harm is a common consequence of mental disorders. This can be especially true for children and young persons, who are often more vulnerable to self-abusive behaviours. Self-harming thoughts can be seeded or encouraged through social interaction with other individuals, particularly those with similar mental disorders or self-harming thoughts. Thoughts about self-harming can also be enhanced by exposure to information relating to self-harming and other mental disorders (for example, news articles, suicidal blogs, etc.). At present, the risk of an individual self-harming is mainly predicated using clinical data. For instance, a strong correlation has been established between suicidal intention and disease types, previous attempts, and ideation history. For the digital generation, clinical correlation alone is not sufficient, since people can be significantly influenced by information from various sources. It is desirable to provide a holistic method to enrich clinical correlation with those from online social network and risks imposed by online information exposure.

In the mental health domain, societal and environmental influence plays a significant role in aggravating and alleviating an individual's situation. The challenge then lies in quantitatively modelling such influence. Such external influence can also be generalised to other lifestyle and wellbeing domains when one's attitude and activities are largely affected and shaped by his/her social interaction and the his/her information consumption. Therefore, solutions addressing the mental illness problem can also be generalised to quantifying societal and environmental factors in other scenarios.

Embodiments of the present invention focus on quantitatively modelling the external influences on an individual to determine the risk of the individual carrying out behaviour demonstrating a mental disorder. This analysis of the external influences on an individual can be used alone or in accordance with other information and factors, such as clinical data.

According to an embodiment of one aspect of the invention, there is provided a risk determining apparatus configured to use data to determine a risk of an individual, suffering a mental disorder, taking self-detrimental action, the apparatus comprising: a data input/output module configured to receive data, relating to a focal individual, from a plurality of data sources; a network analysis module configured to determine a social risk component based on the received data; a text analysis module configured to determine an information risk component based on the received data; and a risk aggregator configured to aggregate the social risk component and the information risk component to determine a numeric risk value.

Thus, the risk of an individual (referred to as a focal individual) carrying out behaviour detrimental to the individual can be determined and quantified using data received from a plurality of data sources that relate to the focal individual. The behaviour may be the consequence or specifically associated with the mental disorder. The risk determining apparatus may determine a risk of the individual self-harming.

The received data is used to determine a social risk component (associated with the focal individual's social contacts) and an information risk component (associated with the focal individual's exposure to the content of relevant information). The social risk component and the information risk component can then be aggregated (combined) to determine a numeric risk value which represents the risk of the focal individual carrying out behaviour that is detrimental to the focal individual. Hence the influence on an individual by information from various sources can be quantitatively modelled, taking into account social context and information content.

Used in the scenario of self-harming, embodiments of the present invention are capable of using data from various sources relating to the social interaction and information consumption of a focal individual, in order to determine whether the focal individual is at risk of committing self-harm. In more general terms, there is a determination of the risk of an individual carrying out behaviour demonstrating a mental disorder using data from various sources which indicate how the individual and their behaviour is influenced by external factors. In many cases, this may be more accurate than using traditional clinical correlation methods. Accordingly, embodiments of the present invention may be used in addition to or as an alternative to traditional clinical correlation methods.

The social risk component may be determined by establishing an ego-centric network of individuals including one or more influencers (influencing individuals) and the focal individual. The ego-centric network can be based on the received data and determine the strength of the one or more of the influencers in the ego-centric network on the focal individual. The ego-centric network may be centred on the focal individual, with the individuals that are influencers formed around the focal individual. The received data from the plurality of data sources may be used to identify influencers in the network and to determine the strength of the influencer on the focal individual (i.e. to what extent the focal individual is influenced by the influencer). Accordingly, the focal individual may be influenced to a greater extent by some influencers compared to others.

Preferably, the strength of the one or more influencers in the ego-centric network on the focal individual is determined for each influencer by weighting interactions between the influencer and the focal individual using network propagation algorithms. Thus, interactions between an influencer and the focal individual may be used to determine the extent to which the influencer is able to influence the focal individual. It may be assumed that an influencer that has many interactions with the focal individual has a greater influence on the focal individual compared to an influencer that only has a small amount of interactions with the focal individual. The interactions used to determine the strength of the influencers can be taken over a given time period and/or through a given means of communication. Additionally or alternatively, interactions over certain time periods (for example, certain times of day) may be weighted differently to interactions at other time periods and interactions through certain means of communication may be weighted differently to interactions through other means.

Each individual in the ego-centric network may be represented by a node, with individuals carrying out behaviour demonstrating a mental disorder having a value of 1 and all other individuals having a value of 0. Initially, all nodes may have a value of 0 and the value of a node may be changed to 1 depending on data received by the data input/output module. An individual may be determined to be carrying out behaviour demonstrating a mental disorder using data received by the data input/output module that relates to the specific individual. The data may be directly obtained from the individual using data sources such as, for example, news, clinical records and/or data related to social media. At this moment, the focal individual may not be involved and so the initial value can be based on known factual data directly, instead of indirect, reference based data. It is preferable that the influence of each node is calculated iteratively and the value at each node is recalculated after each iteration based on the influence propagated by neighbouring nodes. Real-time status updates as to an individual's self-harming activities may be incorporated during run-time, for example during an iteration.

In preferred embodiments, the risk determining apparatus may further comprise a connection to a key term dictionary, with the key term dictionary being configured to store key terms associated with behaviour as a consequence of and/or demonstrating/associated with a mental disorder. The information risk component may then be (at least partially) determined by processing the received data and identifying the significance of key terms in the received data (using the key terms in the key term dictionary). Thus, the received data may be analysed by identifying the occurrence of key terms that are known to be associated with behaviour demonstrating a mental disorder and are stored in the key term dictionary. It is likely that the information risk component will be more accurate if the data is analysed using key terms stored in the dictionary. The key term dictionary may be local or remote and may contain general terms associated with mental health disorders or may include terms relating to specific mental health disorders. This measure of key term significance in the information risk may be of the overall key term appearance in all the documents accessed by the focal individual.

In an embodiment comprising a connection to a key term dictionary, the information risk component may additionally or alternatively perform sentiment analysis on the received data to determine average sentiment values representing the probable perception of documents in the data by the focal individual using the key term dictionary. An individual sentiment value specified for each of the documents in the data is representative of the probable perception of the data by the focal individual, for example, positive, negative or neutral. The average sentiment value is determined using key terms associated with behaviour demonstrating a mental disorder stored in the key term dictionary in combination with the individual sentiment values of the documents.

It is possible that not all key terms contained in the key term dictionary have the same importance or strength of association with behaviour demonstrating a mental disorder and some key terms may be more important than other terms. Accordingly, the information risk component may be adjusted to account for the occurrence of one of more key terms which are more important than other key terms. This comparative weighting of the key terms may be stored in the key term dictionary and used in calculating key term significance and/or overall (average) document sentiment value.

It is preferable that the information risk component for documents accessed by the focal individual combines key term significance with overall document sentiment value. That is, the information risk component from the received data, particularly from documents accessed by the focal individual, may be calculated by combining the determined key term significance with the determined sentiment value. The key term significance may be calculated for all documents accessed by the focal individual (i.e. the entire corpora of documents). Similarly, the document sentiment value may be determined for the entire corpora of documents and then the information risk component may be determined by combining the two figures.

In a preferred embodiment, the risk aggregator is configured to use coefficients refined with time decaying factors to give priority to the most recently received data. The time decaying factors therefore give a higher value to the more recent social and environmental influences, allowing for older influences to gradually fade away as time passes. The use of time decaying factors can ensure that the numeric risk value is representative of the most recent data and is not skewed by old, outdated data.

It is preferable that the risk determining apparatus is further configured to issue a warning, alarm or notification if the numeric risk value passes a predetermined threshold. In some circumstances it may be preferable to issue a warning to a third party if the numeric risk value for the focal individual passes a predetermined threshold. This may indicate that the focal individual is very likely to carry out behaviour as a consequence of a mental disorder so that the notified third party may take appropriate action. In the example case of self-harming or other behaviours as a consequence of a mental disorder, the notification may allow a third party to intervene and prevent the focal individual from causing harm to him or herself. As stated above, the notification/alarm may be triggered when numeric risk value passes a predetermined threshold. Alternatively or additionally, the notification/alarm may be triggered by a sudden rise in the numeric risk value. In other embodiments, the notification/alarm may be triggered by the occurrence of certain activity. The notification/alarm may be used to notify any individual, such as, for example, a medical professional or a friend/relative of the focal individual.

Embodiments of the invention allow for the data input to the system to be continually updated so that the apparatus may use the most recent data. Thus, the risk determining apparatus preferably allows integration of new data during runtime. The new data may be included when determining the social risk component and when determining the information risk component.

The received data may include one or more of: internet browsing history, documents accessed, internet browser log files, social network data, email, text message and call logs. The data relates directly or indirectly to the focal individual and may be collected continually/continuously or over a predetermined period of time.

The data may be collected from one or more computing devices that the focal individual accesses. The devices may include any computing or mobile communications device, such as a mobile telephone, smart phone, tablet, laptop or desktop computer, PDA or other user device.

In some embodiments, the risk aggregator may aggregate the social risk component and the information risk component based on domain knowledge from medical professionals, to provide the numeric risk value normalised with clinical information about the focal individual. The determination of risk may be calculated in accordance with clinical predications and so the numeric risk value may be normalised to account for these predictions. Combining a clinical determination of a focal individual with the methods of the present invention may allow for a more accurate numeric risk value to be determined. Furthermore, the domain knowledge from medical professionals may be predefined and/or input during runtime.

According to an embodiment of a first method aspect of the invention, there is provided a method of using data to determine a risk of an individual, suffering a mental disorder, taking self-detrimental action, the method comprising: receiving data, relating to a focal individual, from a plurality of data sources; determining a social risk component based on the received data; determining an information risk component based on the received data; and aggregating the social risk component and the information risk component to determine a numeric risk value.

The method aspect may be carried out by the same person or commercial entity, or by different people/commercial entities.

According to an embodiment of a further aspect of the invention, there is provided a computer program which, when executed on a computing device, carries out a method of using data to determine a risk of an individual, suffering a mental disorder, taking self-detrimental action, the method comprising: receiving data, relating to a focal individual, from a plurality of data sources; determining a social risk component based on the received data; determining an information risk component based on the received data; and aggregating the social risk component and the information risk component to determine a numeric risk value.

The invention can be implemented in digital electronic circuitry, or in computer hardware, firmware, software, or in combinations of them. The invention can be implemented as a computer program or computer program product, i.e., a computer program tangibly embodied in an information carrier, e.g., in a non-transitory machine-readable storage device or in a propagated signal, for execution by, or to control the operation of, one or more hardware modules. A computer program can be in the form of a stand-alone program, a computer program portion or more than one computer program and can be written in any form of programming language, including compiled or interpreted languages, and it can be deployed in any form, including as a stand-alone program or as a module, component, subroutine, or other unit suitable for use in a communication system environment. A computer program can be deployed to be executed on one module or on multiple modules at one site or distributed across multiple sites and interconnected by a communication network.

Method steps of the invention can be performed by one or more programmable processors executing a computer program to perform functions of the invention by operating on input data and generating output. Apparatus of the invention can be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions and coupled to one or more memory devices for storing instructions and data.

The invention is described in terms of particular embodiments. Other embodiments are within the scope of the following claims. For example, the steps of the invention can be performed in a different order and still achieve desirable results.

The apparatus according to preferred embodiments is described as configured or arranged to carry out certain functions. This configuration or arrangement could be by use of hardware or middleware or any other suitable system. In preferred embodiments, the configuration or arrangement is by software.

Elements of the invention have been described using terms such as "module". The skilled reader will appreciate that these terms and their equivalents may refer to parts of the system that are spatially separate but combine to serve the function defined. Equally, the same physical parts of the system may provide two or more of the functions defined.

Features and sub-features of the system and apparatus aspects may be applied to the method aspects and vice versa

Brief Description of the Drawings

Reference is made, by way of example only, to the accompanying drawings in which:

Figure 1 is an apparatus block diagram representing a risk determining apparatus according to a general embodiment of the invention;
Figure 2 is a flow chart of the method in the risk determining apparatus according to a general embodiment of the invention;
Figure 3 is a graphical representation of an example ego-centric network;
Figure 4 is a block diagram representing system architecture according to an embodiment of an aspect of the invention;
Figure 5 is a flow diagram representing the social influence quantification according to an embodiment of an aspect of the invention;
Figure 6 is a flow diagram representing the information exposure quantification according to an embodiment of an aspect of the invention;
Figure 7 is a block diagram representing a general overview of embodiments of the invention; and
Figure 8 is a block diagram representing a general hardware configuration according to an embodiment of an aspect of the invention.

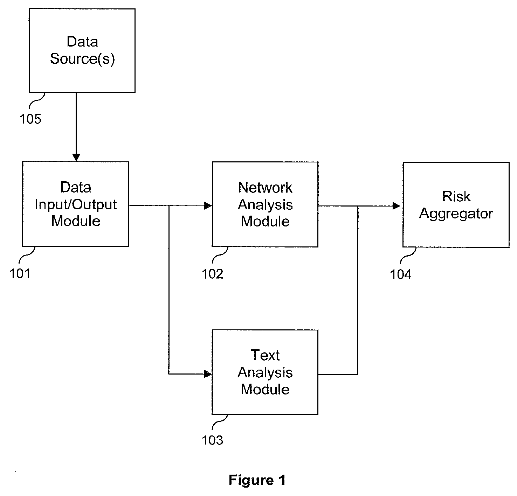

Figure 1 shows a block diagram of a risk determining apparatus according to a general embodiment of the present invention. The risk determination apparatus may be embodied as a PC or another computing apparatus (with data processing capacity) with a GUI and connections via the internet and capable of pulling live data from the internet, such as a server on the internet. It comprises a data input/output module 101, a network analysis module 102, a text analysis module 103 and a risk aggregator 104. The risk determination apparatus also has access to one or more data sources 105 (databases or more simple data stores). The data input/output module 101 may be any device suitable for inputting and/or outputting data and may include, for example, a human computer interface (GUI) that may be used to collect data and/or to present results. The risk determination apparatus may be directly or indirectly connected to the data source(s) 105, through a suitable network connection (for example over the internet). Alternatively or additionally, the risk determination apparatus may comprise one or more local data sources. The data source(s) 105 may contain data from one or more of: internet browsing history, documents accessed, internet browser log files, social network data, email, text message and call logs. The data may be obtained from one or more computing or mobile communications device, such as, for example a mobile telephone, smart phone, tablet, laptop or desktop computer, PDA or other user device. The data may be acquired locally or remotely, in real-time and/or at predetermined intervals.

The risk determining apparatus uses data relating to an individual to determine a risk of the individual, suffering a mental disorder, taking self-detrimental action. The data input/output module receives the data from one or data sources and the data may relate to social and environmental influences associated with the individual. Based on the received data, the network analysis module determines a social risk component and the text analysis module determines an information risk component. The determined social risk and information risk components are aggregated by the risk aggregator to determine a numeric risk value. Thus, the external influences on an individual can be quantitatively modelled to determine a numeric value of the risk of the individual, suffering a mental disorder, taking self-detrimental action.

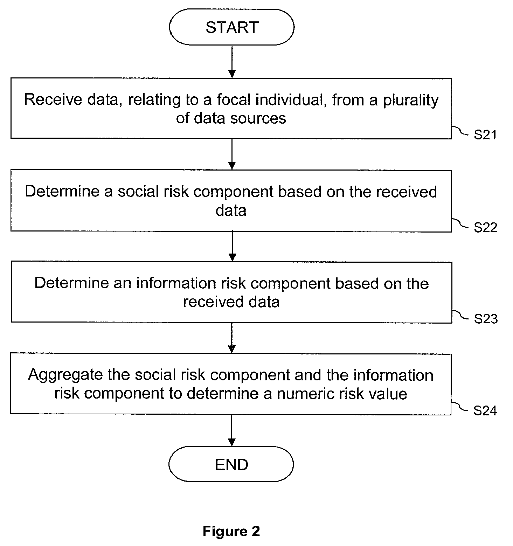

Figure 2 shows a flow chart representing the method in the risk determining apparatus according to a general embodiment of the present invention. Firstly, in step S21, data relating to a focal individual is received from one or more data sources. The received data is then used in step S22 to determine a social risk component and in step S23 to determine an information risk component. In step S24, the social risk component and the information risk component are aggregated in order to determine a numeric risk value, the numeric risk value being indicative of the risk of an individual, suffering a mental disorder, taking self-detrimental action. Steps S22 and S23 may be carried out in the opposite order or simultaneously.

Embodiments of the present invention consider two main types of information: social influence and information exposure.

Social influence

The social niches that individuals reside in can project positive and negative influence on them. It is known in the art that the following factors can have a significant influence on an individual's attitude and intention of carrying out behaviour demonstrating a mental disorder, such as committing self-harm:

- family suicide history;
- social contagion; and
- social isolation.

According to embodiments of the present invention, influence may be modelled through a social network wherein positive and negative influences are propagated along the links connecting individuals.

A typical social network captures individuals and the social connections among them. Social connections are assigned with weights to indicate how strong an influence that one individual can impose on another. Due to data privacy and data safety, such social networks are normally ego-centric networks centred on the focal individual. Initial weights on the focal individual's social connections are assigned as follows: the strength of a social link is evaluated as using the communication frequency between the individuals, including how much time they spend together, how many phone calls they make each day, and how many messages and emails they send to each other. Such information may be collected online through, for example, a consensual sharing of private social network activities, pooling data from the public social network connections, etc., for instance such interaction may be discovered by how frequently one individual replies to or follows messages from another individual, or vice versa.

Note that to precisely define the social interaction is neither possible nor necessary. The social influence model is just to estimate the significance of strong influencers in one's ego-net.

A focal individual's ego-net is defined as a graph, $G_{ego\tau} = \langle N, A \rangle$. The graph consists of a set of nodes $N = \{m\} \cup \{n_0,...,n_n\}$, where m is a graph node representing the focal individual on which the social influence impinges; $n_i$ are m's $\tau$-neighbours. A set of arcs $A$ represents the binary relations over N satisfying the constraint that the length of the shortest path between m and an arbitrary $n_i \in N\{m\}$ should not exceed the threshold $\tau$. When $\tau$ is set to one (i.e. $\tau = 1$), the ego-net $G_{ego1}$ includes only m and its direct neighbours.

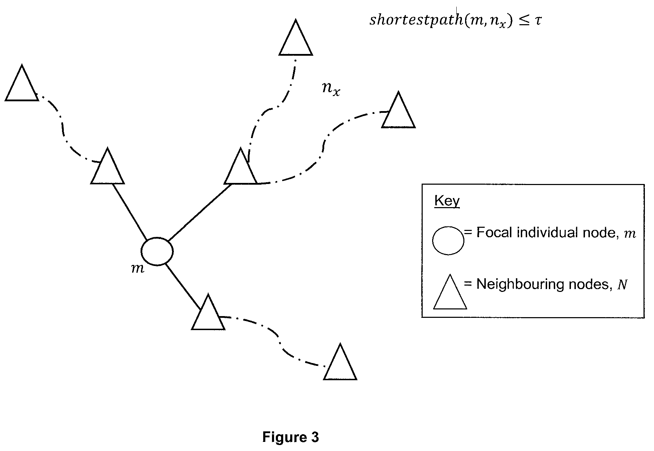

Figure 3 shows a graphical representation of an example ego-centric network. The graph shows a node representing the focal individual, m, surrounded by a number of nodes, N. The nodes $n_0,...,n_i$ are m's $\tau$-neighbours, with a set of arcs showing the path between the focal individual and the neighbour nodes. The ego-centric network includes direct neighbours (indicated by solid lines) and indirect neighbours (indicated by broken lines). To be considered in the network, the neighbours must fall within the shortest path range, $(m, n_x) \leq \tau$.

The social network can be constructed using information from questionnaires, diaries, log files, or through consented access to data dump from social network sites. Data from different sources are considered to be of the same importance. Alternatively, data from some sources may have a higher importance than data from other sources.

A social graph arc (m, n) is weighted based on the number of interactions between the two individuals m and n. The weights are computed using the following heuristics:

1. Emails are counted individually and relative against the total number of emails over a period of time (e.g. day or week). This is formalised as shown in Equation 1 below, where j is the iterator over all contacts of m to give a relative significance of the bound between m and *n.* Time is not explicitly indicated in the formalisation of Equation (1).

$$s_e(m, n) = \frac{\sum from(n) + \sum to(n)}{\sum_{j \in contact(m)} |email(j)|} \tag{1}$$

2. Instant messages are counted per thread and over a period of time, *j*. This is formalised as shown in Equation 2 below.

$$s_m(m, n) = \frac{\sum |msg(n)|}{\sum_{j \in contact(m)} |msg(j)|} \tag{2}$$

3. Voice calls (accessed through the call log) are normalised with the number of calls and the durations of the calls, over a period of time, j. This is formalised as shown in Equation 3 below.

$$s_v(m, n) = \frac{\sum_i call(n, i) \cdot duration(n, i)}{\sum_{j \in contact(m)} \sum_i call(j, i) \cdot duration(j, i)} \tag{3}$$

4. Online activities are counted per thread, over a period of time, j, and with initiators and followers considered separately. This is formalised as shown in Equation 4 below.

$$s_o(m, n) = \frac{1}{2} \left( \frac{a \cdot \sum init(n)}{\sum_{j \in contact(m)} |init(j)|} + \frac{b \cdot \sum follow(n)}{\sum_{j \in contact(m)} |follow(j)|} \right) \tag{4}$$

The weights are normalised over all direct neighbours. Propagation of influence in $G_{ego\tau}$ can be performed based on the standard PageRank model. We assume instead of equal probabilities navigating to all the connected nodes (as stated in the standard PageRank model), the influence imposed by one node on its neighbours are proportional to the weights calculated above. Details of the standard PageRank algorithm are beyond the scope of this proposal, but known to the person skilled in the art.

The initial values of other individuals in the focal individual's social network, $G_{ego\tau}$, are set according to certain behaviour. If there are individuals that are known to carry out behaviour demonstrating a mental disorder, such as self-harm activities, their initial states are set to 1. The initial states are set based on factual information, such as, for example, clinical records. For example, the initial states of all individuals that are known to self-harm or carry out other behaviour as a consequence of a mental disorder are set to 1. At the start of a new social network model, the initial values of all other nodes will be set to 0. At each step of algorithmic iteration, values at each node will be recomputed based on the influence propagated from its neighbouring nodes. The iteration continues until the status change between an iteration and the previous iteration (t + 1 and t) is smaller than a predefined threshold, $\epsilon$, as stated in the standard PageRank algorithm.

The proposed model is dynamic in the sense that real-time status updates are practiced. For instance, whenever a self-harm case is reported or captured, the iteration will be put on hold. A self-harm case may be any recorded self-harm activity, for example, suicide attempts or cases of individuals that have committed suicide. This assessment is mainly based on clinical records and factual information and not on inference or probability. For example, in this case, a web search of the term 'self-harm' would not count as a self-harm activity. Local aggregated influenced values at the nodes representing the individuals having committed self-harm activities (updated through pageRank iterations) will be updated to 1. Further algorithm iterations will be based on the updated status until the converging condition is met.

Thus, in the network of individuals, the initial status is 1 for known self-harm individuals and 0 for others. pageRank will take the links between individuals and their initial status and compute how the influence is passed along the links. At an intermediate stage, the '0's may be updated based on the influence of '1's into a number between 0 and 1.

When the propagation algorithm converges, the aggregated value at m (focal individual node) indicates the influenced likelihood of m to commit self-harm activities. Let the graph of social influence be $G_{\langle S_e, S_m, S_v, S_o \rangle}$, then the societal influence of the i'th individual at iteration t, when the pageRank algorithm converges is shown in Equation 5 below, where $x_i$ is a node or an individual in the social network. This gives the social risk component.

$$soc(x_i^t) = PageRank(G_{\langle S_e, S_m, S_v, S_o \rangle}, x_i^0) \tag{5}$$

Information exposure

Exposure to negative information can also lead to an increased risk of carrying out behaviour demonstrating a mental disorder, such as self-harm. For instance, suicidal ideation is normally seeded through external sources, for example, internet searches, exposure to suicide news stories, etc. Such ideation can be strengthened or weakened through the sentiment of the exposed data. Such factors can be monitored and captured to present a comprehensive numeric value for self-harm risk assessment. Such information exposure is analysed based on the information that focal individuals access on a daily basis. For example, the web pages and other documents that an individual browses and/or the content of emails and messages. The social influence effectively concerns the connection between individuals revealed by, for example, email/messaging and the information exposure concerns the actual contents.

Recently visited information is retrieved using logs from various computing devices that the focal individual accesses. Such documents are then processed with natural language processing techniques to extract key phrases, for example, "suicide methods", "kill myself", etc. Phrases identified as key phrases can be based on a predefined dictionary, based on advice from domain experts and/or associated literature. NLP (neuro-linguistic-programming) techniques are also used to calculate overall sentiment values of each article/document. For more accurate estimation, sentiment values of the documents indicate the potential positive or negative perception that one can receive from such documents. The overall information exposure factor is quantified as discussed below.

Terms identified as 'key terms' are used when analysing the information exposure. The significance of a key term or phrase is quantified using term frequency, normalised against the entire term corpora.

Let D be the dictionary of all self-harm, or a specific set of words and/or phrases, and let d be a term in the dictionary. The adjusted key term value is formalised as shown in Equation 6 below, where C is the corpora of all documents and $c_i$ is a document in the corpora.

$$kt(C) = \frac{1}{|D|} \cdot \sum_{d \in D} \frac{|\{c_i \in C | d \in c_i\}|}{|C|} \tag{6}$$

Equation 6 quantifies the overall key term appearance in the entire corpora of documents, accessed by the individual. This value can be adjusted using the significance of different terms, as shown in Equation 7 below, where $\omega_d$ indicates the significance of term d.

$$kt'(C) = \frac{1}{|D|} \cdot \sum_{d \in D} \frac{\omega_d \cdot |\{c_i \in C | d \in c_i\}|}{|C|} \tag{7}$$

For all the documents, sentiment values are computed using standard, off-the-shelf tools. As public documents are accessed, a generic sentiment repository/dictionary can be used to compute the sentiment value. The overall value is aggregated using term frequency based weights. Again, let D be the dictionary of self-harm words and/or phrases and C be the corpora of all the documents accessed by the individual in the given time period. The adjusted sentiment values are defined as shown in Equation 8 below.

$$avg\text{-}senti(C) = \frac{1}{|C|} \cdot \sum_{c \in C} \left( \frac{\sum_{d \in D} tf(d, c)}{wc(c)} \cdot senti(c) \right) \tag{8}$$

senti(c) gives the overall sentiment value of a document, normally as negative (-1), neutral (0) or positive (+1). In this case, positive is used in the common sense of the word, i.e. a positive document is one that projects a positive feeling. This does not necessarily mean that a document is 'anti' self-harm but that the document is certainly not 'pro' self-harm. tf(d, c) is the term frequency of a given term in a document and wc(c) is the total word count of the document.

Similarly, in order to fine tune the quantitative value, terms defined in D can be weighted by their significance based on, for example, domain expertise. The above computation can then be refined as shown in Equation 9 below.

$$\text{avg-senti}'(C) = \frac{1}{|C|} \cdot \sum_{c \in C} \left( \frac{\sum_{d \in D} \omega_d \cdot \text{tf}(d, c)}{wc(c)} \cdot \text{senti}(c) \right) \qquad (9)$$

The overall information exposure value can then be determined using the key terms and the sentiment value. The overall value can be a simple weighted aggregation of kt(C)/kt'(C) and avg-senti(C)/avg-senti'(C). Note that this value does not present the exact influence of information exposure but rather gives a relative and comparable numeric value that can be combined with other values to signify to what extent the focal individual is at risk of conducting self-harming activities, or other activities that demonstrate a mental disorder.

The overall information value is defined as shown in Equation 10 below, where C is the set of documents that an individual x accessed during a given period of time. This gives the information risk component.

$$\text{info}(x) = kt'(C) \cdot \text{avg-senti}'(C) \qquad (10)$$

The resulting values for the social influence (social risk component) and the information exposure (information risk component) can then be aggregated, as shown in Equation 11 below. This gives the numeric risk value.

$$\text{Risk}(x) = \alpha \cdot \text{soc}(x) \cdot e^{-\lambda t_1} + \beta \cdot \text{info}(x) \cdot e^{-\lambda t_2} \qquad (11)$$

Coefficients $\alpha$ and $\beta$ are refined with time decaying factors to give higher values to the most recent social and environmental influences, which then gradually feed away as time passes. The overall non-clinical risk value may also need to be normalised so as to combine with clinical predictions or domain knowledge provided by experts.

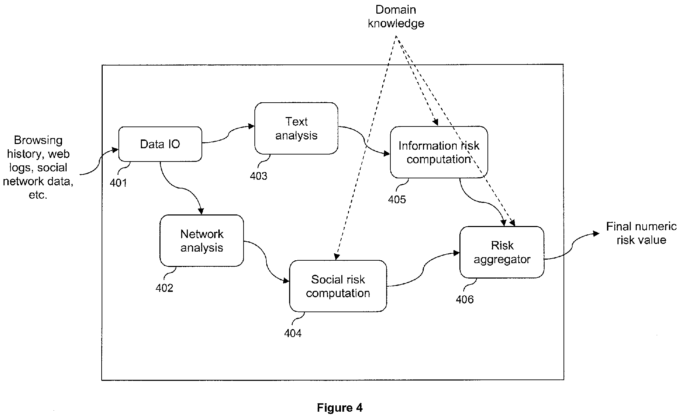

Figure 4 is a block diagram representing system architecture according to an embodiment of an aspect of the invention. Data IO 401 is responsible for collecting and performing initial cleansing/preprocessing of data from users browsing history, internet browser log files, and social network data that are consented for sharing. The collected data is then fed into the network analysis component 402 and the text analysis (with NLP capacity) component 403 for social influence quantification 404 and information exposure quantification 405, respectively. The results from these two analytics components 404, 405 are aggregated using dedicated aggregators. The overall numeric risk value is computed using the final risk aggregator 406. Aggregation models rely on domain knowledge from experts which can be either predefined or input at run-time according to system prompts.

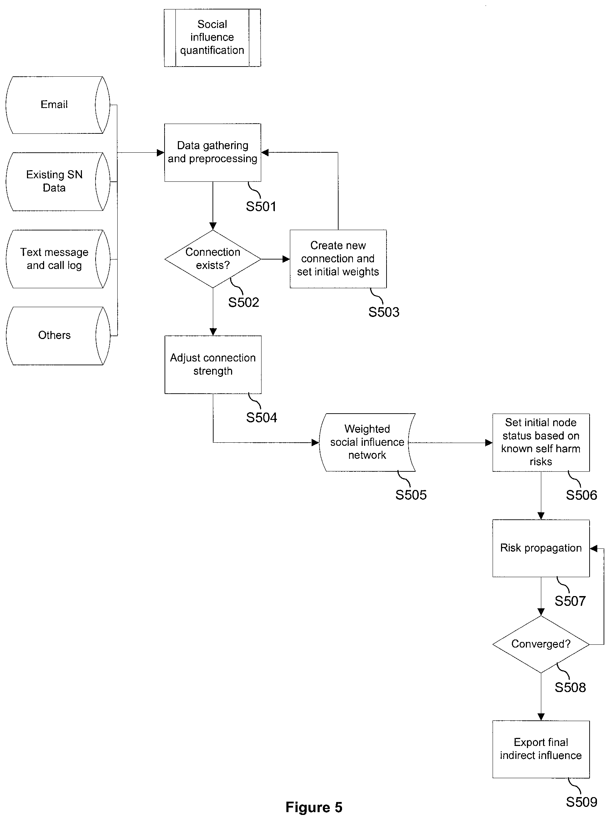

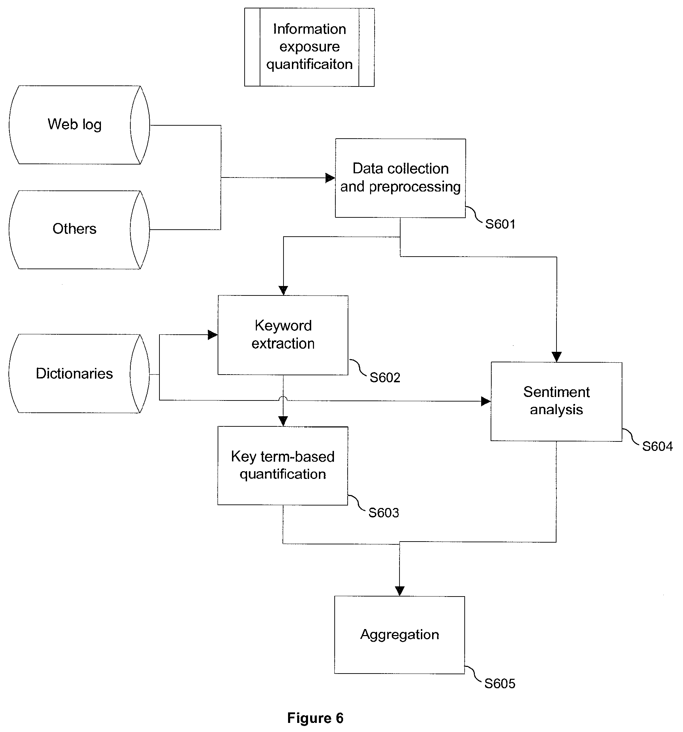

The process will be discussed, by way of example only, with reference to Figures 5 and 6.

The social influence quantification effectively includes two processes: the social network construction and connection strength adjustment and known self-harm risk propagation. The first process collects data from various sources to construct a person's social network (either online, using existing social network data on a consent basis or offline, using email, SMS, call logs, or other available sources). The basic idea is to establish an n-hop ego-centric network so as to understand how an individual can be influenced by others that he or she may contact and have some faith in. The second process is to understand the strength of the influence. By using existing network propagation algorithms, this process tries to quantify how a self-harm activity in one's social network can impinge on the focal individual.

Figure 5 is a flow diagram representing the social influence quantification according to an embodiment of an aspect of the invention. In step S501, the data is gathered from various sources and preprocessed. The data is used to determine connections between a focal individual and other individuals, and to create the ego-centric network. When other individuals are detected from the data, a connection is made between the focal individual and the detected individual. In step S502 it is determined whether a connection between the individuals has been established in the network. If not, the process proceeds to step S503 where the new connection is created and initial weights are set. If the connection has already been established, the process proceeds to step S504, in which the connection strength is adjusted according to the data and interactions between the individuals. In step S505, the weighted social influence network is established to conclude the first half of the social influence quantification process.

In step S506, the initial values of the nodes are set based on known self-harm risks. The risk propagation method, such as the pageRank algorithm is then run and the values of the nodes are updated accordingly in step S507. This continues until the propagation algorithm converges in step S508 and the final indirect social influence is exported in step S509.

The information exposure quantification may also be considered as two separate processes. The first process focuses on keyword/key term based quantification, which highlights words or phrases that are directly associated with self-harm activities. The second process is to understand the general sentiment value of the information accessed by the focal individual.

Figure 6 is a flow diagram representing the information exposure quantification according to an embodiment of an aspect of the invention. Data is collected from various sources and preprocessed in step S601. In step S602, the key words are extracted from the data using one or more dictionaries defining the key terms and the key term analysis is quantified in step S603. Sentiment analysis is conducted in step S604 and also uses the dictionaries defining key terms. The resultant values from the key term analysis and the sentiment value analysis are then aggregated in step S605 to give the final information exposure value.

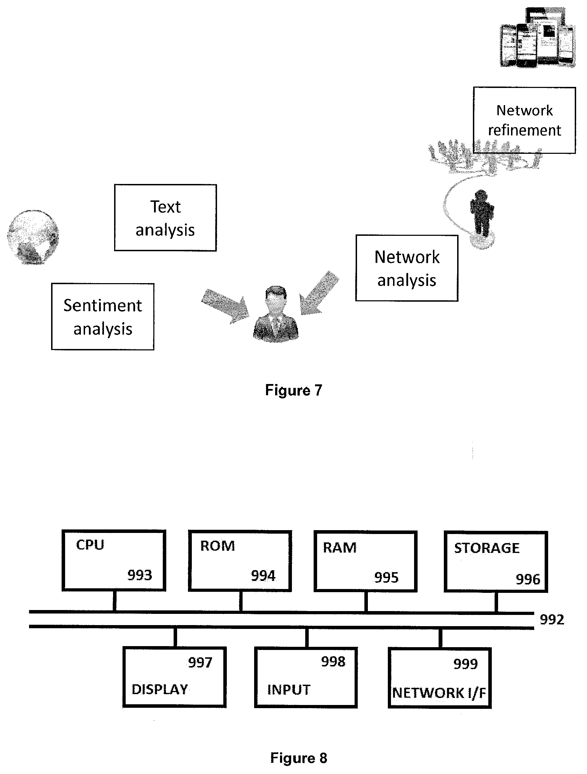

Figure 7 is a block diagram representing a general overview of embodiments of the invention and shows the combination of the text analysis 701 and the sentiment analysis 702 which make up the information exposure quantification (information risk component), along with the network analysis 703 which provides the social influence quantification (social risk component). The network analysis 703 is based on network refinement 704 of an individual and their influences.

Figure 8 is a block diagram of a computing device, in this case a data storage server, which is programmed to embody the present invention, and which may be used to implement a method of an embodiment and to provide the data input/output module, network analysis module, text analysis module and risk aggregator as described herein. The computing device comprises a computer processing unit (CPU) 993, memory, such as Random Access Memory (RAM) 995, and storage, such as a hard disk, 996. The computing device also includes a network interface 999 for communication with other computing devices, potentially also invention embodiments. For example, an embodiment may be composed of a network of such computing devices. Optionally, the computing device also includes Read Only Memory 994, one or more input mechanisms such as keyboard and mouse 998, and a display unit such as one or more monitors 997. The components are connectable to one another via a bus 992.

The CPU 993 is configured to control the computing device and execute processing operations. The RAM 995 stores data being read and written by the CPU 993. The storage unit 996 may be, for example, a non-volatile storage unit, and is configured to store data. The display unit 997 displays a representation of data stored by the computing device and displays a cursor and dialog boxes and screens enabling interaction between a user and the programs and data stored on the computing device. The input mechanisms 998 enable a user to input data and instructions to the computing device.

The network interface (network I/F) 999 is connected to a network, such as the Internet, and is connectable to other such computing devices via the network. The network I/F 999 controls data input/output from/to other apparatus via the network. Other peripheral devices such as microphone, speakers, printer, power supply unit, fan, case, scanner, trackerball etc may be included in the computing device.

Methods embodying the present invention may be carried out on a computing device such as that illustrated in Figure 8. Such a computing device need not have every component illustrated in Figure 8, and may be composed of a subset of those components. A method embodying the present invention may be carried out by a single computing device in communication with one or more data storage servers via a network. The computing device may be a data storage server itself. A method embodying the present invention may be carried out by a plurality of computing devices operating in cooperation with one another. One or more of the plurality of computing devices may be a data storage server.

As discussed in detail, the final evaluation is based on two separate types of data, which are aggregated and may be adjusted using time factors. The proposed method can be used to quantify external influence that impinges (either encouragingly or discouragingly) on one's intention or ideation of self-harm activities, or other behaviour that is a consequence of a mental disorder. This method, subject to minor adjustment, can be applied to other similar situations, in which social and information exposure can influence one's decision making and when such external factors need to be quantitatively evaluated.

The proposed method does not necessarily present a comprehensive risk assessment of self-harm activities and the results can complement clinical ones to give more accurate estimation, situating the focal individuals in the social niche.

Invention embodiments may:

  1. Present a clear mechanism to perform quantitative analysis on

      a. How an individual's intention to carry out behaviour demonstrating a mental disorder (for example, self-harm) is or could be influenced by his/her online social network.
      b. How one is exposed to information and how they perceive that information

  2. Offer a dynamic and real-time risk assessment based on the information accessed by a focal individual.

**Claims**

1. A risk determining apparatus configured to use data to determine a risk of an individual, suffering a mental disorder, taking self-detrimental action, the apparatus comprising:

   a data input/output module configured to receive data, relating to a focal individual, from a plurality of data sources;
   a network analysis module configured to determine a social risk component based on the received data;
   a text analysis module configured to determine an information risk component based on the received data; and
   a risk aggregator configured to aggregate the social risk component and the information risk component to determine a numeric risk value.

2. A risk determining apparatus according to claim 1, wherein the self-detrimental action is action that is a consequence of the mental disorder, such as self-harming.

3. A risk determining apparatus according to any preceding claim, wherein the network analysis module is configured to determine the social risk component by establishing an ego-centric network of individuals including one or more influencers and the focal individual, the ego-centric network being based on the received data and determining the strength of the one or more of the influencers in the ego-centric network on the focal individual; and preferably wherein the strength of the one or more influencers in the ego-centric network on the focal individual is determined for each influencer by weighting interactions between the influencer and the focal individual using network propagation algorithms.

4. A risk determining apparatus according to claim 3, wherein each individual in the ego-centric network is represented by a node, with an individual carrying out behaviour demonstrating a mental disorder having a value of 1 and all other individuals having a value of 0; and preferably wherein the influence of each node is calculated iteratively and the value at each node is recalculated after each iteration based on the influence propagated by neighbouring nodes.

5. A risk determining apparatus according to any preceding claim, further comprising a connection to a key term dictionary, the key term dictionary being configured to store key terms associated with behaviour demonstrating a mental disorder, and wherein the text analysis module is configured to determine the information risk component by processing the received data to identify the significance of key terms in the received data.

6. A risk determining apparatus according to any preceding claim, further comprising a connection to a key term dictionary, the key term dictionary being configured to store key terms associated with behaviour demonstrating a mental disorder, and wherein the text analysis module is configured to determine the information risk component by performing sentiment analysis on the received data to determine sentiment values representing the probable perception of documents in the data by the focal individual using the key term dictionary.

7. A risk determining apparatus according to claim 5 or 6, wherein the information risk component is adjusted to account for the occurrence of one or more key terms which are more important than other key terms; and/or wherein the information risk component for documents accessed by the focal individual combines key term significance with overall document sentiment value.

8. A risk determining apparatus according to any preceding claim, wherein the risk aggregator is configured to use coefficients refined with time decaying factors to give priority to the most recently received data.

9. A risk determining apparatus according to any preceding claim, further comprising a notification module configured to issue a warning, alarm or notification if the numeric risk value passes a predetermined threshold.

10. A risk determining apparatus according to any preceding claim, wherein
    the data input/output module is configured to allow integration of new data during runtime;
    the network analysis module is configured to include the new data when determining the social risk component; and
    the text analysis module is configured to include the new data when determining the information risk component.

11. A risk determining apparatus according to any preceding claim, wherein the received data includes one or more of:

internet browsing history, documents accessed, internet browser log files, social network data, email, text message and call logs.

12. A risk determining apparatus according to any preceding claim, wherein the input/output module is configured to receive data from one or more computing devices that the focal individual accesses.

13. A risk determining apparatus according to any preceding claim, wherein the risk aggregator is configured to aggregate the social risk component and the information risk component based on domain knowledge from medical professionals, to provide the numeric risk value normalised with clinical information about the focal individual; and preferably wherein the domain knowledge is predefined and/or input during runtime.

14. A method of using data to determine a risk of an individual, suffering a mental disorder, taking self-detrimental action, the method comprising:

> receiving data, relating to a focal individual, from a plurality of data sources;
> determining a social risk component based on the received data;
> determining an information risk component based on the received data; and
> aggregating the social risk component and the information risk component to determine a numeric risk value.

15. A computer program which, when executed on a computing device, carries out a method of using data to determine a risk of an individual, suffering a mental disorder, taking self-detrimental action, the method comprising:

> receiving data, relating to a focal individual, from a plurality of data sources;
> determining a social risk component based on the received data;
> determining an information risk component based on the received data; and
> aggregating the social risk component and the information risk component to determine a numeric risk value.

Figure 1

START

Receive data, relating to a focal individual, from a plurality of data sources
S21

Determine a social risk component based on the received data
S22

Determine an information risk component based on the received data
S23

Aggregate the social risk component and the information risk component to determine a numeric risk value
S24

END

Figure 2

$$shortestpath(m, n_x) \leq \tau$$

Key

◯ = Focal individual node, $m$

△ = Neighbouring nodes, $N$

**Figure 3**

**Figure 4**

**Figure 5**

Figure 6

Figure 7

Figure 8

Europäisches
Patentamt

European
Patent Office

Office européen
des brevets

# EUROPEAN SEARCH REPORT

Application Number

EP 16 17 0786

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 660 745 A2 (ALMOSNI BERNIE [IL]; TAL ILAN [IL]) 6 November 2013 (2013-11-06) * paragraph [0006] - paragraph [0054] * ----- | 1-15 | INV. G06F19/00 G06Q30/00 |
| A | MUNMUN DE CHOUDHURY ET AL: "Predicting postpartum changes in emotion and behavior via social media", HUMAN FACTORS IN COMPUTING SYSTEMS, ACM, 2 PENN PLAZA, SUITE 701 NEW YORK NY 10121-0701 USA, 27 April 2013 (2013-04-27), pages 3267-3276, XP058043170, DOI: 10.1145/2470654.2466447 ISBN: 978-1-4503-1899-0 * the whole document * ----- | 1-15 | |
| A | Munmun De Choudhury ET AL: "Predicting Depression via Social Media", AAAI Conference on Artifical Intelligence and interactive Digital Entertainment, 1 July 2013 (2013-07-01), XP055317502, Retrieved from the Internet: URL:http://course.duruofei.com/wp-content/ uploads/2015/05/Choudhury_Predicting-Depre ssion-via-Social-Media_ICWSM13.pdf [retrieved on 2016-11-08] * the whole document * ----- | 1-15 | **TECHNICAL FIELDS SEARCHED (IPC)** G06F G06Q |
| A | US 8 214 253 B1 (HARRIS DAVID LINDEL [US] ET AL) 3 July 2012 (2012-07-03) * the whole document * ----- -/-- | 1-15 | |

The present search report has been drawn up for all claims

| Place of search | Date of completion of the search | Examiner |
|---|---|---|
| The Hague | 15 November 2016 | Schechner-Resom, M |

EPO FORM 1503 03.82 (P04C01)

Europäisches
Patentamt

European
Patent Office

Office européen
des brevets

# EUROPEAN SEARCH REPORT

Application Number

EP 16 17 0786

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TINGSHAO ZHU ET AL: "Predicting Mental Health Status Based on Web Usage Behavior", 7 September 2011 (2011-09-07), ACTIVE MEDIA TECHNOLOGY, SPRINGER BERLIN HEIDELBERG, BERLIN, HEIDELBERG, PAGE(S) 186 - 194, XP019161895, ISBN: 978-3-642-23619-8 * the whole document * | 1-15 | |
| A | US 2014/201126 A1 (ZADEH LOTFI A [US] ET AL) 17 July 2014 (2014-07-17) * the whole document * | 1-15 | |
| A | MUNMUN DE CHOUDHURY ET AL: "Inferring relevant social networks from interpersonal communication", PROCEEDINGS OF THE 19TH INTERNATIONAL CONFERENCE ON WORLD WIDE WEB, WWW '10, 1 January 2010 (2010-01-01), page 301, XP055318202, New York, New York, USA DOI: 10.1145/1772690.1772722 ISBN: 978-1-60558-799-8 * the whole document * | 1-15 | |

TECHNICAL FIELDS
SEARCHED      (IPC)

The present search report has been drawn up for all claims

| Place of search | Date of completion of the search | Examiner |
|---|---|---|
| The Hague | 15 November 2016 | Schechner-Resom, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 17 0786

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2016

| Patent document cited in search report | | Publication date | Patent family member(s) | | Publication date |
|---|---|---|---|---|---|
| EP 2660745 | A2 | 06-11-2013 | EP<br>US | 2660745 A2<br>2013297536 A1 | 06-11-2013<br>07-11-2013 |
| US 8214253 | B1 | 03-07-2012 | US<br>US | 8214253 B1<br>8645187 B1 | 03-07-2012<br>04-02-2014 |
| US 2014201126 | A1 | 17-07-2014 | NONE | | |

EPO FORM P0459

For more details about this annex : see Official Journal of the European Patent Office, No. 12/82